Europäisches Patentamt

European Patent Office

Office européen des brevets

(11) Publication number: **0 556 521 A1**

# EUROPEAN PATENT APPLICATION

(21) Application number: **92311875.6**

(22) Date of filing: **31.12.92**

(51) Int. Cl.⁵: **C12N 1/06**, //C12N15/00, C12Q1/68

(30) Priority: **09.01.92 US 819353**

(43) Date of publication of application:
**25.08.93 Bulletin 93/34**

(84) Designated Contracting States:
**DE ES FR GB IT SE**

(71) Applicant: **Becton Dickinson and Company
One Becton Drive
Franklin Lakes New Jersey 07417-1880(US)**

(72) Inventor: **Dey, Margaret Sigler
7920 Secluded Acres Road
Apex, North Carolina 27502(US)**
Inventor: **Keating, Edward William
500 Woodcroft Parkway
No. 18-A, Durham, North Carolina 27713(US)**
Inventor: **Siddiqi, Salman ul-Haq
15 Glencoe Manor Court
Sparks, Maryland 21152(US)**
Inventor: **Down, James Arthur
101 Charter Oaks Circle
Cary, North Carolina 27511(US)**

(74) Representative: **Ruffles, Graham Keith et al
MARKS & CLERK 57-60 Lincoln's Inn Fields
London WC2A 3LS (GB)**

(54) **Sample processing using disinfectant.**

(57) The invention is a process for lysing a microorganism which comprises exposing the microorganism to a lysis effective amount of disinfectant.
The process provides several advantages including simplicity, rapidity, and safety.

EP 0 556 521 A1

Rank Xerox (UK) Business Services
(3.10/3.6/3.3.1)

Field of the Invention

The invention is in the field of molecular biology. In particular the invention is in the area of cell lysis.

Background of the Invention

Intracellular components are essential for a variety of reasons. Intracellular components such as DNA, RNA, proteins, proteoglycans, carbohydrates, and lipids are useful for a variety of detection events.

To obtain intracellular components the cell of interest must be opened. Methods to obtain intracellular components include the use of sonication, pH extremes, detergents, chaotropes, solvents, grinding, cavitation, and osmotic shock.

Known processes for obtaining intracellular components include U.S. patent 4,830,969 which discloses a process for isolation of nucleic acids involving lysis buffer, heating, and purification, EPO 393,744 which discloses methods for detecting nucleic acid from blood or peripheral blood mononulear cell (PBMC) fractions which involves heating the fraction at or near the boiling point of water, U.S. patent 4,923,978 which discloses a process for purifying nucleic acid which involves passing a solution of DNA and protein over hydroxylated support to bind protein, U.S. patent 4,908,318 which discloses a nucleic acid extraction involving buffy coat, protease, and perchlorate and isopropanol precipitation of DNA, U.S. patent 4,900,677 which discloses a process for isolation of high molecular weight DNA involving lysis with detergent, RNA degradation, protein denaturation with chaotropes, and DNA dialyzed and concentrated, and A. Brisson-Noel et al., The Lancet Nov 4:1069 (1989) which discloses a procedure for diagnosis of Mycobacterium tuberculosis which uses sodium hydroxide, sodium chloride, and sodium dodecylsulphate (SDS).

It is desirable to have a process for obtaining intracellular components which is rapid and simple. In addition, the intracellular components should be capable of detection, amplification, and the like.

Summary of the Invention

The invention is a process for lysing a microorganism which comprises exposing the microorganism to a lysis effective amount of disinfectant.

The process provides several advantages including simplicity, rapidity, and safety. Not only are intracellular components liberated by practicing the process of the invention, but the sample is also rendered safe for handling by the addition of a disinfectant. Safety is a major concern when handling samples suspected of containing pathogenic organisms. In addition, the process of the invention is applicable to all microorganisms, ranging from the easily lysed microorganisms to the most difficult.

As used in this document, "Sample" refers to the assimilation of any substance from any source with or without containment means. Sample typically refers to fluid, tissue, and the like obtained from blood, serum, mucus, sputum, and the like, from a patient that is human or a veterinary patient. "Lysis effective amount" refers to that necessary to liberate the desired intracellular component from a sample, but which amount does not destroy or render the intracellular components unsuitable for subsequent use.

Detailed Description of the Invention

The use of a disinfectant to render a microorganism noninfectious and lysed is surprising. For example, one would expect a disinfectant to destroy or render the DNA unuseable due to the harsh chemical nature of disinfectants. However, by using a disinfectant in accordance with the invention, DNA is released that can subsequently be used in amplification protocols, detection protocols, and the like.

The use of disinfectants at too high of concentrations can render the DNA unuseable. The appropriate concentration of disinfectant is readily determined by applying an amount to a sample and evaluating the DNA obtained. After an appropriate concentration of disinfectant is determined for the liberation of DNA from the particular sample, a series of samples can be easily run.

All disinfectants are by definition bacteriocidal. However, not all disinfectants produce sufficient bacterial lysis that is compatible with subsequent DNA amplification and/or detection. The addition of a lysis-effective disinfectant to a sample enables both the lysis of the organisms and renders the sample safe (i.e. non pathogenic). Otherwise, a sample may be rendered safe by disinfectant treatment and lysis can otherwise be obtained with a variety of lytic agents comprising enzymes and the like.

Although disinfectants such as O-SYL, ROCCAL II, VESPHENE, and HIBICLENS have been sucessfully employed in the practice of the invention, other disinfectants can also be used. Preferably ranges are from about 1/2% to about 10%. Preferably the ranges for CHLOROX are from about 1/2% to about 8%, with about 1% most preferred. Preferred ranges for STAPHENE are from about 1/2% to about 10%, with about 5% most preferred. LYSOL preferred ranges are from about 1/2% to about 10%, with about 3% most preferred.

The format of the organism from which the intracellular components are to be obtained is not crucial to the invention. The organism can be cultured or in a clinical sample. Sources of samples can come from unpurified biological samples like sputum, feces, tissue, blood, serum, tissue, other body fluids and the like. Bacteria obtained from the various sources are typically cultured, which is very time consuming, reaching three to six weeks culture time (especially in the case of Mycobacteria). However, by practicing the method of the invention, the requirement to culture is eliminated. If culturing is not desired, the cells are generally first isolated from the source by conventional sample processing methods then usually pelleted by centrifugation and put into a cell suspension. The ability to use the process of the invention with a clinical sample is particularly advantageous.

If the sample is a sputum sample it is generally liquified by a variety of procedures prior to adding a disinfectant. For example, one suitable protocol for liquification of sputum samples can be found in Kubica et al., American Review of Respiratory Diseases 87:775 (1963), incorported herein by reference. After liquification, the sample is usually neutralized, then separated to obtain a pellet fraction, which contains the desired organism. To the pellet fraction is added a disinfectant. If the sample does not need to be liquified, disinfectant may be added immediately.

After disinfection is substantially complete, the organisms are collected, typically in the form of a pellet. Preferably a brief incubation period is warranted in order for the disinfectant to act. The pellet can then be used in DNA binding protocols for separating the DNA with subsequent elution and isolation of DNA. Conventional methods for isolating DNA include phenol:chloroform extractions, glass binding with subsequent elution, and the like. Examples of conventional protocols for isolating DNA are found in references such as T. Maniatis et al., Molecular Cloning: A Laboratory Manual (Cold Spring Harbor Lab) (1982) and Boom et al., J. Clin. Micro **28**:495 (1990), both incorporated herein by reference. DNA purification procedures also include Celite purification procedures as disclosed in Volgelstein and Gillespie, Proc. Nat. Acad. Sci. U.S.A. 76:615 (1979), Willis et al., Biotechniques 9:92 (1990), and McCormick, Analytical Biochemistry 181:66 (1989), all incorporated herein by reference. When the DNA is obtained in its desired form and purity, subsequent amplification protocols, detection protocols, and the like can be employed.

For example, typical purification steps for obtaining DNA from a lysed sample include organic extractions such as phenol/chloroform extractions. Suitable amplification procedures include polymerase chain reaction (PCR), PCR Technology, H.A. Erlich, Ed. (Stockton Press, New York, NY, 1989), transcription-based amplification system (TAS), Proc. Natl. Acad. Sci. USA **86**:1173 (1989), ligation amplification reaction (LAR), Genomics **4**:560 (1989), ligase based amplification system (LAS), Gene **89**:117 (1990), and Q B replicase, Infect. Dis. **162**:13 (1990).

Identification and location of intracellular components and microorganisms can take place by means of a variety of identifying agents. Identifying agents refers to those agents suitable for identifying microorganisms and components which include nucleic acid probes including deoxyribonucleic acid and ribonucleic acid, and the like. The presence of a particular microorganism can then be detected by a variety of means, depending on the marker (e.g., signal to be detected) chosen for use with the identifying agent. The means for identification of the presence of a component or microorganism is usually dictated by the identifying agent employed. For example, nucleic acid probes (e.g., specific for a Mycobacteria species) are typically labeled with $^{125}$I, $^{32}$P, fluorescent markers, and the like. The marker is then detected, which detection is an indication that the particular substance is present. Other means for detection include Southern Blot analysis, electrophoretic gel visualization, and the like.

The disinfectants can be conveniently provided in the form of a kit. Such a kit can further comprise at least one type of identifying agent and a lysis effective amount of disinfectant. Specific kits can comprise identifying agents for any microorganism or component for a specific microorganism. Specific kits also can comprise particular identifying means such as nucleic acid probes or antibodies. And, the means by which the identifying agent is detected can also be specific, for example, the agent can be designed for fluorescence, radioactive, and chemiluminescence detection and, if necessary, depending on sample requirements, liquification agents, isolation agents, and the like can be included in the kit. Further embodiments of the kits comprise lysis effective amounts of reagents such as achromopeptidase, and the like.

The process of the invention is not limited to use with any particular organism. Organisms such as Mycobacteriacea, which are considered extremely difficult to obtain intracellular components from, are suitable for use with the process of the invention. Other organisms include but are not limited to those from the family Acetobacteraceae, Legionellaceae, Neisseriacea, Flavobacterium, Enterobacteriaceae, Vibrionaceae, Pasteurellaceae, Bacteroidaceae, Veillonellaceae, Rickettsiaceae, Bartonellaceae, Anaplasmatacae, Chlamydiaceae, Mycoplasmataceae, Spirochaetaceae, and Pseudomonadaceae.

Important bacteria that can be lysed by practicing the present invention include Mycobacteria, such as M. avium, M. intracellularae, M. gordonae, M. tuberculosis, M. kansasii, M. fortuitum, M.chelonae, M. bovis, M. scrofulaceum, M. paratuberculosis, M. marinum, M. simiae, M. szulgai, M. intracellulare, M. xenopi, M. ulcerans, M. leprae, M. lepraemurium, M. smegmatis, M. flavescens, M. terrae, M. nonchromogenicum, M. malmoense, M. asiaticum, M. vaccae, M. gastri, M. triviale, M. haemophilum, M. africanum, M. thermoresistable, and M. phlei. Several of the Mycobacteria are pathogenic. For example, M. tuberculosis, which already infects two billion people and infects an additional seven to nine million people each year, is an important Mycobacteria from an epidemiologic and clinical viewpoint In addition, M. avium, M. bovis, M. intracellularae, M. africanum, M. leprae, M. chelonae, M. paratuberculosis, and M. marinum, are also significant from an epidemiological and clinical viewpoint.

The following examples illustrate the specific embodiments of the invention described in this document. As would be apparent to skilled artisans, various changes and modifications are possible and are contemplated within the scope of the invention described.

EXAMPLE 1

Materials:

STAPHENE concentrate (Calgon, St. Louis, Mo.) LYSOL (Nat'l Labs Inc., Montvale, NJ EPA #T7303C 164P) White Arrow Bleach (Astor Products, Jacksonville, FL) 10% SDS Stock Solution (BRC Cat #585UGA Lot 9W302) BACTEC (Becton, Dickinson and Co., Towson, MD) AMPLITAQ (Cetus Perkin Elmer, Norwalk CT)

Mycobacterium tuberculosis culture bottle

Heat inactivated at 68° C 4 hours H37 RV strain (American Type Culture Collection (ATCC)) at BACTEC G.I. >999

Procedure:

8 M. tuberculosis "pellets" were created as follows:

1 ml of culture was removed from the BACTEC bottle using a syringe and placed into an Eppendorf 1.5 ml centrifuge tube. The samples were centrifuged for 5 minutes. Then the supernatant was aspirated using a syringe and discarded. An additional 1 ml wash was used, with centrifugation and aspiration repeated.

The pellets were treated as follows:

Using 6 different disinfectants; either 1% or 10% bleach, 1% or 5% STAPHENE (v/v) or 3% or 10% LYSOL (v/v), each pellet was reconstituted in 500 $\mu$l of the disinfectant and incubated at room temperature for 1 hour. After this, half of each sample was subjected to 2 phenol/chloroform extractions and 1 chloroform extraction was performed,
followed by ethanol precipitation. Each sample was reconstituted to 100 $\mu$l with sterile $H_2O$. Half of each sample was further incubated with 1% SDS for 15 minutes then treated as above. 50 $\mu$l of each product was used in a PCR reaction mixture containing IS6110 (Eisenach et al. Nucleic Acid Res. **18**:188 (1990) and Gene Bank Accession No. M298-99) bases 956-981 and bases **1024**-1050 with 2.5 units tag polymerase per test.

The other 50 $\mu$l was dried overnight to a pellet, then reconstituted into 10 $\mu$l $H_2O$ plus 3$\mu$l tracking dye. These samples were run on a 1% agarose gel in TAE (40 mM Tris acetate 1 mM EDTA) buffer. The conditions for the thermal cycler are as follows:

| | |
|---|---|
| 5 minutes | 95° C |
| 1 minute (30 cycles) | 95° C |
| 2 minutes (30 cycles) | 70° C |
| 7 minutes | 72° C |
| - | 4° C |

10 $\mu$l of PCR product was removed and added to 3 $\mu$l loading dye, then run on a 10% acrylamide gel.

All gels were ethidium bromide stained (.5 $\mu$g/ml) 10 minutes and visualized under UV light.

Conclusions:

Disinfectants can extract amplifiable amounts of M. tuberculosis DNA in addition to lysis as evidenced by detection of the amplified 74 base pair IS6110 target sequence on ethidium bromide stained 10% polyacrylamide gels.

By combining the SDS with the disinfectant, we may get increased lysis, but the DNA is no longer amplified by PCR.

EXAMPLE 2

Procedure:

2 ml pellets of M. tuberculosis were produced as previously described

Each of seven pellets were redissolved into 500 $\mu$l of one of the following buffers:

| #1 | 1% bleach |
|----|-----------|
| #2 | 10% bleach |
| #3 | 5% STAPHENE |
| #4 | 10% LYSOL |
| #5 | 1% SDS |
| #6 | Phenol/chloroform (50/48/2) |
| #7 | Tween 80 (1%)(detergent used as Standard) |

Each sample was incubated at room temperature for 30 minutes with occasional vortexing, except #5 which was incubated in boiling water for 15 minutes, then incubated at room temperature for 15 minutes.

To each sample, was added 500 $\mu$l of phenol/chloroform/Isoamyl Alcohol (50:48:2), except #6, to which was added 500 $\mu$l H$_2$O.

Samples were spun down and the aqueous layer was extracted again.

The sample with 1% SDS was extracted a third time, since the aqueous phase was still cloudy after 2 extractions.

The 1% Tween sample was extracted 4 times (to clarify the aqueous phase).

All samples were subjected to a chloroform/Isoamyl alcohol extraction (using equal volumes) then ethanol precipitated in 2 volumes 70% ETOH and 1/10 volume 3M sodium acetate by -20° C for 1 hour and -70° C for 15 minutes.

The dried pellet was resuspended into 100 $\mu$l sterile H$_2$O.

PCR reaction mixes were set up using 50 $\mu$l of stock PCR reaction mixture and 50 $\mu$l of sample. The mixture contained the IS6110 primer set (bases 956-981 of IS6110 and bases 1024-1050 of IS6110). Also run was a 50 $\mu$l negative control consisting of water and a positive control consisting of 10 $\mu$l of 50$\mu$g/ml SK 4.3 plasmid (a plasmid containing the IS6110 sequence) (500 pg). Samples were thermal cycled according to the previously mentioned protocol.

10 $\mu$l of each product was combined with 4$\mu$l tracking dye and electrophoresed on a 10% acrylamide gel. The gel was then ethidium bromide stained and photographed. The remainder of the samples were tested in blot hybridization reactions for ability to bind to a $^{32}$P-labeled, M. tuberculosis-specific DNA probe (see example 5).

The ethidium-stained gel showed the following samples to contain the highly stained bands representing high amplification of the amplified 74 base pair IS6110 target sequence:

10% bleach

5% STAPHENE

10% LYSOL

Tween 80 (1%) (Sigma, St, Louis, MO)

Positive Control (500 picograms of plasmid SK4.3 which contains the whole IS6110 sequence)

Slight contamination of the negative is also seen in 2 sizes. At ~ 74 bp and also ~ 60 bp.

The phenol/chloroform treatment alone produced a very weakly amplified product. Thus the enhanced PCR amplification observed for the above treatments is due to improved lysis and results in increased release of the target sequence.

EXAMPLE 3

This example sets forth procedures demonstrating amplification with naked DNA (i.e., not lysed and then amplified).

Materials:

Five Clinical Samples (cervical swabs) spiked with M. tuberculosis in $H_2O$
Milli Q $H_2O$ (Millipore Corp., Bedford, MA) non-sterile
Sterile Milli Q $H_2O$
SK4.3 plasmid (plasmid that contains the IS6110 sequence)
SK4.3 primers Sequence I.D. No: 3 and 4
$\beta$-actin + control ($\beta$actin positive control is a $\beta$actin target sequence supplied by Clontech (cat. no. 5402-1)-)
$\beta$-actin Primers Sequence I.D. No: 1 and 2
Tag Polymerase
PCR mix
Sterile TE (10mM Tris, 1mM EDTA)
Parafin Oil
Diatom Suspension
Diatom Isolation Reagents
Loading Dye (25% Ficoll 400, .25% Bromophenol blue, .25% Xylene Cyanol)
10% acrylamide gels
10 X TBE (0.89 M Tris-Borate, 0.01M EDTA)
MWM (molecular weight markers) (Biomarker™ low molecular weight standards from Bioventures, Inc., Murfreesburo, TN)
Ethidium Bromide (Sigma, St. Louis, MO)
Polaroid 57 and 55 film (Cambridge, MA)
1% agarose gel in TAF
MWM for 1% agarose gel (Lamda phage DNA restricted with HIND III restriction Endonuclease from Bethesda Research Labs (cat. no. 56125A))

Disinfectants:

Hypochlorite (bleach) (White Arrow - Astor Products Inc.    EPA est #51790-GA-1)
STAPHENE (Calgon Corp. Merch EPA est #1043-MO-1)
LYSOL (National LaboratorieS EPA est #675-19
SDS - 10% stock BRL Cat #5553UA Lot #9PW302)
Tween 80 (Fisher Scientific T-164 Lot #713715)
VESPHENE II (Vestal Products Lot #31HO02H9)
O-SYL (National Labs Lehn & Fink Prod. EPA #T3048C)
AMPHYL (National Labs Lehn & Fink Prod EPA #T9263C1644)
CIDEX-PLUS (Surgikos, Arlington, TX)
HIBICLENS (Stuart Pharmaceuticals, Wilmington, DE)
GLUTAREX (3M, St. Paul, MN)
ROCCAL II (National Labs AB8430 EPA #T4138C)
Tween 80 (Fisher Scientific T-164 Lot #713715) was used as a standard.

Procedure:

Each disinfectant was made in 10 ml aliquots at 10% or by manufacturers' instructions as follows:.

| | |
|---|---|
| Bleach | 1 ml bleach 9 ml tap $H_2O$ |
| AMPHYL | 1ml in 9ml $H_2O$ |
| LYSOL | 1ml in 9ml $H_2O$ |
| SDS | 10% stock |
| HIBICLENS | 1ml in 9ml $H_2O$ |
| Tween 80 | 1ml in 9ml $H_2O$ |
| VESPHENE II | 78 $\mu$l into 10 ml $H_2O$ (1:128) |
| ROCCAL II | 156 $\mu$l into 10 ml $H_2O$ (1:64) |
| O-SYL | 78 $\mu$l into 10 ml $H_2O$ (1:128) |
| STAPHENE | 78 $\mu$l into 10 ml $H_2O$ (1:128) |
| GLUTADEX | 434 $\mu$l activator to 10 ml diluent (1:23) |
| CIDEX-PLUS | 400 $\mu$l activator to 10 ml diluent |

The fluid from all 5 clinical samples was combined, the swabs were each washed by vortexing in .5 ml $H_2O$ and combined with initial sample fluid. Each sample and wash was about 2 mls, making the total volume ~10ml.

50$\mu$l of SK4.3 plasmid (5 ng/$\mu$l) was added and sample vortexed.

From the pooled sample were made 19 (.5 ml) aliquots which were centrifuged in an Eppendorf centrifuge for 2 minutes and supernatants discarded.

One pellet was set aside as an untreated control.

The remaining 18 pellets were resuspended in .5 ml of one of the test disinfectants at a given concentration:

| Tube No. | Disinfectant | Concentration |
|---|---|---|
| 1 | BLEACH | 1% |
| 2 | BLEACH | 5% |
| 3 | AMPHYL | 1% |
| 4 | AMPHYL | 5% |
| 5 | LYSOL | 1% |
| 6 | LYSOL | 5% |
| 7 | SDS | 1% |
| 8 | SDS | 5% |
| 9 | HIBICLENS | 1% |
| 10 | HIBICLENS | 5% |
| 11 | TWEEN 80 | 1% |
| 12 | TWEEN 80 | 5% |
| 13 | VESPHENE II | According to Manufacturer's specs |
| 14 | ROCCAL III | According to Manufacturer's specs |
| 15 | O-SYL | According to Manufacturer's specs |
| 16 | STAPHENE | According to Manufacturer's specs |
| 17 | GLUTADEX | According to Manufacturer's specs |
| 18 | CIDEX-PLUS | According to Manufacturer's specs |

All samples were incubated for 30 minutes at room temperature with the exception of the SDS samples which were boiled for 15 minutes then incubated at room temperature for 15 minutes.

Samples were centrifuged 2 minutes in eppendorf centrifuge and supernatants discarded.

The untreated pellet (which was set aside) was processed with all the others through the diatom isolation/purification protocol.

Samples were eluted in 100 $\mu$l (50 + 50 $\mu$l) and total volume was brought up to ~ 100 $\mu$l with sterile $H_2O$ to account for pellet sample absorbtion.

The following PCR reaction mix was made up consisting of 1050 $\mu$l PCR buffer, 10.5 $\mu$l Tag Polymerase, 21 $\mu$l each primers Sequence I.D. No: 1 and 2, and 21 $\mu$l each SK4.3 primers Sequence I.D. No: 3 and 4.

10 $\mu$l of each sample was mixed with 40 $\mu$l sterile $H_2O$.

Positive control of $\beta$-actin plus control (1 $\mu$l) + SK4.3 + control (5 $\mu$l) was made up with 44$\mu$l sterile $H_2O$.

Negative control was 50$\mu$l sterile $H_2O$.

50$\mu$l of PCR reaction mix was added to each sample and control.

All tubes were overlayed with parafin oil and subjected to PCR.

To analyze the PCR products, 10$\mu$l of each sample was mixed with 2$\mu$l loading dye and run on a 10% acrylamide gel at 100 v for ~ 40 minutes. Running buffer was 1 X TBE.

Gels were stained with Ethidium Bromide, 15 minutes, backstained in milli Q $H_2O$ for 15 minutes and photographed under ultraviolet light with Polaroid 57 film (1 second) and Polaroid 55 film (10 second). Negatives of 55 films were developed for densitometry readings.

Results

Analysis of the gels containing the ethidium-stained PCR products indicated that the following detergents did not inhibit PCR amplification of the actin or IS6110 targets: O-SYL, ROCCAL II, VESPHENE, HIBICLENS. The following disinfectant treatments were compatible with PCR (i.e. an amplified product was observed) but produced less amplification than the control reaction: AMPHYL, LYSPL, GLUTADEX, and CIDEX. It was also noted that 1% Tween 80 slightly increased PCR amplification of either target, whereas bleach and SDS were inhibitory (i.e., little or no product was observed).

Active Ingredients:

|  |  |
|---|---|
| Tween 80 | (Tween 80) |
| O-SYL | (o-benzyl-p-Chlorophenol, Isopropanol, o-phenylphenol) |
| ROCCAL II | (Ethanol, dimethyl benzlammonium chloride, Alkyl $C_{14}$ 50%/$C_{12}$ 40%/$C_{10}$ 10%) |
| VESPHENE | Na-orthophenylphenate, Na-o-benzl-p-Chlorophenate, NA-p-tertiam amgaphorate |
| HIBICLENS | 4% hibitane (chlorhexidine gluconate) |

The disinfectants and detergents tested were grouped into four categories: 1. agents which enhance PCR amplification; 2. those best for PCR (of the agents tested); 3. agents which are compatible with PCR; and 4. those which inhibit PCR.

| 1. ENHANCE | 2. BEST | 3. COMPATIBLE | 4. INHIBITORY |
|---|---|---|---|
| Tween 80(1%) | HIBICLENS<br>Tween 80<br>VESPHENE<br>ROCCAL II<br>O-SYL | AMPHYL<br>LYSOL<br>GLUTADEX<br>CIDEX | Bleach<br>SDS |

Only the detergent Tween 80 1% seems to aid in sample processing. Those disinfectants or detergents listed in the Best or Compatible column did not interfere to a great degree in sample target amplification. They were also the same processes which showed the greatest amount of sample DNA on an agarose gel, as well as Tween 80 1%. Although they do not aid in sample processing, they do not interfere to a great degree.

Conclusion:

Several treatments inhibited target amplification while others interfered little - showing possibility for use in safer sample handling while leaving the DNA able to be amplified.

EXAMPLE 5

Purpose: To determine the effect of disinfectant treatment on PCR amplification of a DNA target (as for example 3).

Materials:

Disinfectants and other materials as for example 3.

Procedure:

500 $\mu$l of the following solutions were placed in 1.5 ml Eppendorf tubes.

| | | | |
|---|---|---|---|
| 1. | VESPHENE (1:128) | 11. | HIBICLENS (5%) |
| 2. | ROCCAL II (1:128) | 12. | SDS (1%) |
| 3. | O-SYL (1:128) | 13. | SDS (5%) |
| 4. | STAPHENE (1:200) | 14. | TWEEN 80 (1%) |
| 5. | AMPHYL (1:200) | 15. | TWEEN 80 (5%) |
| 6. | GLUTAREX (1:23)* | 16. | TWEEN 20 (1%) |
| 7. | CODEX-PLUS (1:25)* | 17. | TWEEM 20 (5%) |
| 8. | BLEACH (1%) | 18. | LYSOL (1%) |
| 9. | BLEACH (5%) | 19 | LYSOL (5%) |
| 10. | HIBICLENS (1%) | | |

*ratio of activator to diluent

10 $\mu$l of linearized SK 4.3 plasmid DNA (equivalent to 100 targets) was added to each solution, vortexed, then incubated at room temperature for 10 minutes.

The samples were microfuged for 3 minutes and the supernatants were discarded.

Each pellet was resuspended in 100 $\mu$l sterile $H_2O$, from which 10 $\mu$l was added to a further 40 $\mu$l sterile $H_2O$ and subjected to PCR amplification.

The positive control consisted of 10 $\mu$l untreated stock solution of plasmid in 40 $\mu$l sterile $H_2O$. The negative control consisted of 50 $\mu$l of sterile $H_2O$.

10 $\mu$l of each PCR product was analyzed on an ethidium-stained 10% polyacrylamide gel.

Result

The greatest amounts of amplified product were observed in the VESPHENE and O-SYL treatments. Fainter bands were observed in the 1% LYSOL STAPHENE, AMPHYL, HIBICLENS and TWEEN treatments. No amplification was observed after treatments with ROCCAL II, GLUTAREX, CIDEX PLUS.

EXAMPLE 4

In this experiment, the DNA from disinfectant-lysed M. tuberculosis (i.e., 1% CHLOROX, 5% STAPHENE, 3% LYSOL) is clearly visualized by hybridization to a radioactive probe and subsequent autoradiography.

MATERIALS AND METHODS

As described in Example 2.
PROBE - bases 140-220 of IS6110

DISCUSSION

Disinfectants Chlorox, Staphene, and Lysol produced DNA that gave the best hybridization signals. A higher concentration of Chlorox (i.e. 10% versus 1%) resulted in loss of signal. The various lysis procedures that were tested gave blot hybridization results which varied from negative (in the case of the Sjobring and DeWit methods) to strongly positive (as for lyticase and achromopeptidase). Intermediate results were obtained with the following disinfectants: 1% Chlorox, 5% Staphene, and 3% Lysol which produced better hybridization than the two published lysis methods of A. Sjobring et al., J. of Clin.Microbiol **28**:2200 (1990) and D. DeWit et al., J. of Clin Microbiol **28**:2437 (1990). The data show that i) disinfectants release amplifiable DNA from M. tuberculosis and ii) the disinfectant process does not denature the DNA.

EXAMPLE 6

Purpose: To determine the effect of disinfectants on DNA liberated from M. tuberculosis by boiling.

Materials:

Disinfectants and other materials as for example 3.

Procedure:

Dilutions of disinfectants were made up as described in experiment 5.

1 ml of a suspension of heat-inactivated BACTEC-cultured M. tuberculosis organisms was removed aseptically by syringe from the BACTEC bottle and diluted in $H_2O$ to $10^5$/ml. 100 $\mu$l of this suspension was added to 500 $\mu$l of each disinfectant and incubated at room temperature for 10 minutes.

The samples were microfuged for 3 minutes, the supernatants discarded and the pellets resuspended in 10 mM Tris-HCl, 1 mM EDTA containing 1% Triton X-100.

The samples were then boiled for 10 minutes to release the M. tuberculosis DNA from the organisms.

5 $\mu$l of each sample was combined with 45 $\mu$l sterile $H_2O$ and subjected to PCR according to the cycling protocol outlined in example 1.

10 $\mu$l of each PCR product was analyzed on an ethidium-stained 10% polyacrylamide gel.

Result

Brightly fluorescent bands of amplified target, indicative of high amplification, were observed after treatment with all of the agents except bleach or 5% SDS.

EXAMPLE 7

Purpose: To determine the effect of disinfectants on DNA liberated from M. tuberculosis by treatment with proteolytic enzymes.

Materials:

Disinfectants and other materials as for example 3.

Procedure:

Disinfectants and detergents were diluted into 500 $\mu$l aliquots as described in example 6. Each aliquot received $10^4$ M. tuberculosis organisms as described in example 6.

Each reaction was vortexed, incubated at room temperature for 10 minutes, then microfuged for 3 minutes. Supernatants were discarded and each pellet was resuspended in 400 $\mu$l of sterile $H_2O$, to which was added 50 $\mu$l Proteinase K (10mg/ml), 50$\mu$l 0.1 M EDTA, and 18 $\mu$l Achromopeptidase (10 mg/ml).

Reactions were incubated at 50°C for 1 hour, then microfuged for 3 minutes and supernatants discarded.

DNA from each reaction was isolated by adsorption to diatoms according to published protocol (Boom et al, 1990, J. Clin. Micro **28(3)**:495-503.

1/10 of the DNA eluted from the diatoms (i.e., 10 $\mu$l of 100 $\mu$l eluate) was combined with 40 $\mu$l sterile $H_2O$ and subjected to PCR amplification as described in example 1.

Result:

Brightly flourescent bands of amplified target, indicative of high amplification, was observed after the following treatments: CIDEX-PLUS, HIBICLENS, 5% SDS. Less amplification occurred after treatment with any of the other agents. After no treatment there was observed a lack of amplification.

SUMMARY OF EXAMPLES 5 - 7

| Lysis Method: | UNPROTECTED DNA | PROTECTED DNA | |
| --- | --- | --- | --- |
| | | (i.e. inside organisms) | |
| | None | Boiling | Proteases |
| AGENT | | | |
| VESPHENE | + | +++ | + |
| ROCCAL II | − | +++ | + |
| O−SYL | + | +++ | + |
| STAPHENE | + | +++ | + |
| AMPHYL | + | +++ | + |
| GLUTAREX | − | +++ | + |
| CIDEX−PLUS | − | +++ | ++ |
| BLEACH (1%) | − | +/− | + |
| BLEACHJ(5%) | − | +/− | + |
| HIBICLENS (1%) | + | ++ | ++ |
| HIBICLENS (5%) | + | ++ | +++ |
| LYSOL (1%) | + | +++ | + |
| LYSOL (5%) | − | +++ | + |
| Control | + | +++ | ++ |

Legend: −, no amplified target observed; +/−, a faint band observed; +, clearly visible band of amplified product; ++, a strongly fluorescent band indicative of good amplification; +++, a very highly fluorescent band indicative of a very high level of amplification.

Note: Columns represent examples 5 −7 respectively and results should be compared relative to the positive control, which is an independent indicator of how well the PCR reaction ran between experiments.

CONCLUSIONS FROM EXAMPLES 5 - 7

Each type of target (i.e., unprotected M. tuberculosis DNA versus DNA released from M. tuberculosis organisms by boiling or proteolysis) has a specific set of disinfectants with which it is most compatible, as evidenced by subsequent PCR amplification. No disinfectant was completely compatible to all three types of target (i.e., produced an amplified target to the same level or greater than each respective control reaction). Thus, a choice of disinfectant for a specific application depends upon the nature of the target DNA to be amplified.

Overall Conclusion

Use of disinfectants accomplished two functions in a single step: disinfection of the sample and liberation of intact DNA.

Although the invention has been described with respect to specific modifications, the details thereof are not to be construed as limitations, for it will be apparent that various equivalents, changes and modifications may be resorted to without departing from the spirit and scope thereof, and it is understood that such equivalent embodiments are to be included therein.

SEQUENCE LISTING

(1) GENERAL INFORMATION:

    (i) APPLICANT: Down, James A
                 Keating, William E
                 Dey, Margaret S
                 Siddiqi, Salman U

    (ii) TITLE OF INVENTION: Sample Processing Using Disinfectant

    (iii) NUMBER OF SEQUENCES: 4

    (iv) CORRESPONDENCE ADDRESS:
          (A) ADDRESSEE: Richard J. Rodrick
          (B) STREET: 1 Becton Drive
          (C) CITY: Franklin Lakes
          (D) STATE: New Jersey
          (E) COUNTRY: U.S.A.
          (F) ZIP: 07417-1880

    (v) COMPUTER READABLE FORM:
          (A) MEDIUM TYPE: Floppy disk
          (B) COMPUTER: IBM PC compatible
          (C) OPERATING SYSTEM: PC-DOS/MS-DOS
          (D) SOFTWARE: PatentIn Release #1.0, Version #1.25

    (vi) CURRENT APPLICATION DATA:
          (A) APPLICATION NUMBER:
          (B) FILING DATE:
          (C) CLASSIFICATION:

   (viii) ATTORNEY/AGENT INFORMATION:
          (A) NAME: Stierwalt, Brian K
          (B) REGISTRATION NUMBER: 33,213
          (C) REFERENCE/DOCKET NUMBER: P-2233

    (ix) TELECOMMUNICATION INFORMATION:
          (A) TELEPHONE: 201-847-5317
          (B) TELEFAX: 201-848-9228

(2) INFORMATION FOR SEQ ID NO:1:

    (i) SEQUENCE CHARACTERISTICS:
          (A) LENGTH: 24 base pairs
          (B) TYPE: nucleic acid
          (C) STRANDEDNESS: single

```
        (xi) SEQUENCE DESCRIPTION: SEQ ID NO:1:

GACAACGGCT CCGGCATGTG CAAG                                        24

(2) INFORMATION FOR SEQ ID NO:2:

        (i) SEQUENCE CHARACTERISTICS:
            (A) LENGTH: 24 base pairs
            (B) TYPE: nucleic acid
            (C) STRANDEDNESS: single


        (xi) SEQUENCE DESCRIPTION: SEQ ID NO:2:

TACCTGGTGC CTGGGGCGCC CCAC                    .                    24

(2) INFORMATION FOR SEQ ID NO:3:

        (i) SEQUENCE CHARACTERISTICS:
            (A) LENGTH: 25 base pairs
            (B) TYPE: nucleic acid
            (C) STRANDEDNESS: single


        (xi) SEQUENCE DESCRIPTION: SEQ ID NO:3:

GACCCGCCAA CAAGAAGGCG TACTC                                       25

(2) INFORMATION FOR SEQ ID NO:4:

        (i) SEQUENCE CHARACTERISTICS:
            (A) LENGTH: 25 base pairs
            (B) TYPE: nucleic acid
            (C) STRANDEDNESS: single


        (xi) SEQUENCE DESCRIPTION: SEQ ID NO:4:

ATGTGTACTG AGATCCCCTA TCCGT                                       25
```

The preferred disinfectants include not only the formulated materials identified by the Trade Mark designations, but also more generally any disinfectants which contain the active disinfectant ingredient(s) present in such formulated materials. Such active disinfectant materials are suitably employed at concentrations comparable to those applicable for the respective formulated materials identified by the Trade Mark designations.

**Claims**

1. A process for lysing a microorganism which comprises exposing the microorganism to a lysis effective amount of disinfectant.

2. The process of claim 1 in which the disinfectant is selected from the group consisting of O-SYL, ROCCAL II, VESPHENE, CHLOROX, STAPHENE, LYSOL, and HIBICLENS.

3. The process of claim 2 in which the disinfectant is from about 1/2% to about 10%.

4. The process of claim 3 in which the disinfectant is O-SYL.

5. The process of claim 3 in which the disinfectant is ROCCAL II.

6. The process of claim 1 in which the microorganism is selected from the group consisting of Mycobacteriacea, Acetobacteraceae, Legionellaceae, Neisseriacea, Flavobacterium, Enterobacteriaceae, Vibrionaceae, Pasteurellaceae, Bacteroidaceae, Veillonellaceae, Rickettsiaceae, Bartonellaceae, Anaplasmatacae, Chlamydiaceae, Mycoplasmataceae, Spirochaetaceae, and Pseudomonadaceae.

7. The process of claim 6 in which the Mycobacteria is selected from the group consisting of M. avium, M. intracellularae, M. gordonae, M. tuberculosis, M. Kansasii, M. fortuitum, M. chelonae, M. bovis, M. scrofulaceum, M. paratuberculosis, M. phlei, M. marinum, M. simiae, M. scrofulaceum, M. szulgai, M. intracellulare, M. leprae, M. xenopi, M. ulcerans, M. lepraemurium, M. flavescens, M. terrae, M. nonchromogenicum, M. malmoense, M. asiaticum, M. vaccae, M. gastri, M. triviale, M. haemophicum, M. africanum, M. thermoresistable, and M. smegmatis.

8. The process of claim 7 in which the Mycobacteria is M. tuberculosis.

9. A kit comprising a lysis effective amount of disinfectant.

10. The kit of claim 33 in which the disinfectant is selected from the group consisting of O-SYL, ROCCAL II, VESPHENE, CHLOROX, STAPHENE, LYSOL, and HIBICLENS.

14

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 83, no. 83, 4 August 1975, Columbus, Ohio, US; abstract no. 38196v, A.R.W. SMITH ET AL. 'DIFFERING EFFECTS OF CETYLTRIMETHYLAMMONIUMBROMIDE AND CETRIMIDE B.P. UPON GROWING CULTURES OF ESCHERICHIA COLI NCIB 8277' page 93 ;column 2 ; * abstract * & J. APPL. BACTERIOL. vol. 38, no. 2, 1975, pages 143 - 9 --- | 1 | C12N1/06 //C12N15/00 C12Q1/68 |
| X | FR-A-1 491 224 (CANADIAN PATENTS AND DEVELOPMENT LTD) * Summary. 1; 2a,f * * page 2, column 1, paragraph 3 * * page 1, column 2, paragraph 1 * --- | 1-3,5,6, 9,10 | |
| X | DE-A-3 337 566 (ROHTO PHARMACEUTICAL CO) 19 April 1984 * page 6; claims 1,2; example * --- | 1-3,5 | TECHNICAL FIELDS SEARCHED (Int. Cl.5) |
| X | DE-A-1 470 190 (RYSTAN COMPANY MOUNT VERNON) 10 April 1969 * claims; examples 1,2,6,8,11,14 * * page 5, paragraph 2 * * page 6, paragraph 2 * * page 9, paragraph 2 * * page 11, paragraph 1 * * page 12, paragraph 2 * * page 13, paragraph 2 * * page 14, paragraph 1 * --- -/-- | 1-3,5,9, 10 | C12N |

The present search report has been drawn up for all claims

| Place of search | Date of completion of the search | Examiner |
|---|---|---|
| THE HAGUE | 28 APRIL 1993 | COUCKE A.O.M. |

EPO FORM 1503 03.82 (P0401)

European Patent
Office

EUROPEAN SEARCH REPORT

Application Number

EP    92 31 1875
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 043 475 (LABORATOIRES CASENNE) 19 February 1971 * claims 1,5,7,9-11 * * page 5 * * page 8; example 1 * --- | 1,6,9 | |
| X | FR-A-580 481 (M. KAHN) * summary 1,2 * * page 1, line 10 - page 2, line 29 * --- | 1,9,10 | |
| X | EP-A-0 398 677 (PRESIDENT AND FELLOWS OF HARVARD UNIVERSITY) 22 November 1990 * column 6, line 3 - line 16; claims 1,8,9 * --- | 1,6-8,9 | |
| X | SUSAN BUDAVARI ET AL. 'THE MERCK INDEX' 1989 , MERCK &CO INC , USA * p.165, nr 1066; p323, nr 2090; p. 1363, nr.8576 * ----- | 1-3,5,9, 10 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 28 APRIL 1993 | COUCKE A.O.M. |